# EUROPÄISCHE PATENTSCHRIFT

(11) **EP 1 531 770 B1**
(45) Veröffentlichungstag und Bekanntmachung des Hinweises auf die Patenterteilung: **05.07.2017**
(21) Anmeldenummer: 03750427.1
(22) Anmeldetag: 22.08.2003
(51) Int. Cl.: A61F 9/008

(54) **VORRICHTUNG ZUR MESSUNG EINES OPTISCHEN DURCHBRUCHS IN EINEM GEWEBE**
DEVICE FOR MEASURING AN OPTICAL PENETRATION IN A TISSUE
DISPOSITIF DE MESURE D'UNE OUVERTURE OPTIQUE DANS UN TISSU

(30) Priorität: 23.08.2002 DE 10239213; 23.05.2003 DE 10323422
(43) Veröffentlichungstag der Anmeldung: 25.05.2005
(60) Teilanmeldung: 17172442.0
(73) Patentinhaber: Carl Zeiss Meditec AG, 07745 Jena (DE)
(72) Erfinder: MÜHLHOFF, Dirk, 07751 Kunitz (DE); KEMPE, Michael, 07751 Kunitz (DE); GERLACH, Mario, 16548 Glienicke-Nordbahn (DE); STICKER, Markus, 07749 Jena (DE); BISCHOFF, Mark, 07749 Jena (DE); DICK, Manfred, 07926 Gefell (DE); STREHLE, Markus, 07751 Jena (DE); BERGT, Michael, 94425 Weimar (DE)
(74) Vertreter: Patentanwälte Geyer, Fehners & Partner mbB
(86) Internationale Anmeldenummer: PCT/EP2003/009345
(87) Internationale Veröffentlichungsnummer: WO 2004/026198

(56) Entgegenhaltungen:
- WO-A-01/80792
- WO-A-01/91661
- DE-C- 19 635 998
- JUHASZ T ET AL: "TIME-RESOLVED OBSERVATIONS OF SHOCK WAVES AND CAVITATION BUBBLES GENERATED BY FEMTOSECOND LASER PULSES IN CORNEAL TISSUE AND WATER" LASERS IN SURGERY AND MEDICINE, WILEY- LISS, NEW YORK, US, Bd. 19, Nr. 1, 1996, Seiten 23-31, XP000597102 ISSN: 0196-8092

## Beschreibung

Die Erfindung bezieht sich auf eine Vorrichtung zur Messung eines optischen Durchbruches, der in einem Gewebe unterhalb einer Gewebeoberfläche von einer Behandlungs-Laserstrahlung ausgelöst wird, die eine laser-chirurgische Einrichtung in einem im Gewebe liegenden Behandlungsfokus bündelt, wobei die Vorrichtung einen Detektionsstrahlengang mit einer Optik aufweist. Die Erfindung bezieht sich weiter auf ein Verfahren zur Messung eines optischen Durchbruchs, der in einem Gewebe unterhalb einer Gewebeoberfläche von Behandlungs-Laserstrahlung ausgelöst wird.

Behandlungs-Laserstrahlung wird in laserchirurgischen Verfahren insbesondere für augenchirurgische Eingriffe angewendet. Dabei wird die Behandlungs-Laserstrahlung innerhalb des Gewebes, d.h. unterhalb der Gewebeoberfläche, derart fokussiert, daß ein optischer Durchbruch im Gewebe ausgelöst wird. Die Behandlungs-Laserstrahlung wirkt durch Photodisruption oder -ablation.

Im Gewebe laufen zeitlich hintereinander mehrere Prozesse ab, die durch die Behandlungs-Laserstrahlung initiiert werden. Überschreitet die Leitungsdichte der Strahlung einen Schwellwert, kommt es zu einem optischen Durchbruch, der im Gewebe eine Plasmablase erzeugt. Diese Plasmablase wächst nach Entstehen des optischen Durchbruches durch sich ausdehnende Gase. Wird der optische Durchbruch nicht aufrechterhalten, so wird das in der Plasmablase erzeugte Gas vom umliegenden Gewebe aufgenommen, und die Blase verschwindet wieder. Dieser Vorgang dauert allerdings sehr viel länger, als die Entstehung der Blase selbst. Wird ein Plasma an einer Gewebegrenzfläche erzeugt, die durchaus auch innerhalb einer Gewebestruktur liegen kann, so erfolgt ein Gewebeabtrag von der Grenzfläche. Man spricht deshalb dann von Photoablation. Bei einer Plasmablase, die vorher verbundene Gewebeschichten trennt, ist üblicherweise von Photodisruption die Rede. Der Einfachheit halber werden all solche Prozesse hier unter dem Begriff optischer Durchbruch zusammengefaßt, d.h. dieser Begriff schließt nicht nur den eigentlichen optischen Durchbruch sondern auch die daraus resultierenden Wirkungen im Gewebe ein.

Für eine hohe Genauigkeit eines laserchirurgischen Verfahrens ist es unumgänglich, eine hohe Lokalisierung der Wirkung der Behandlungs-Laserstrahlen zu gewährleisten und Kollateralschäden in benachbartem Gewebe möglichst zu vermeiden. Es ist deshalb im Stand der Technik üblich, die Behandlungs-Laserstrahlung gepulst anzuwenden, so daß der zur Auslösung eines optischen Durchbruchs nötige Schwellwert für die Leistungsdichte der Behandlungs-Laserstrahlung nur während den einzelnen Pulsen überschritten wird. Die US 5.984.916 zeigt diesbezüglich deutlich, daß der räumliche Bereich des optischen Durchbruchs (in diesem Fall der erzeugten Wechselwirkung) stark von der Pulsdauer abhängt. Eine hohe Fokussierung des Laserstrahls in Kombination mit sehr kurzen Pulsen erlaubt es damit, den optischen Durchbruch sehr punktgenau in einem Gewebe einzusetzen.

Der Einsatz von gepulster Behandlungs-Laserstrahlung hat sich in der letzten Zeit besonders zur laserchirurgischen Fehlsichtigkeitskorrektur in der Ophthalmologie durchgesetzt. Fehlsichtigkeiten des Auges rühren oftmals daher, daß die Brechungseigenschaften von Hornhaut und Linse keine ordnungsgemäße Fokussierung auf der Netzhaut bewirken. Bei Kurzsichtigkeit (auch als Myopie bezeichnet) liegt bei entspanntem Auge der Fokus vor der Netzhaut, bei Fernsichtigkeit (auch als Hyperopie bezeichnet) ist der Fokus dagegen hinter der Netzhaut.

Die erwähnte US 5.984.916 sowie die US 6.110.166 beschreiben Verfahren zur Fehlsichtigkeitskorrektur mittels geeigneter Erzeugung optischer Durchbrüche, so daß im Endeffekt die Brechungseigenschaften der Hornhaut gezielt beeinflußt werden. Eine Vielzahl von optischen Durchbrüchen wird so aneinander gesetzt, daß innerhalb der Hornhaut des Auges ein linsenförmiges Teilvolumen isoliert wird. Das vom übrigen Hornhautgewebe getrennte linsenförmige Teilvolumen wird dann mittels eines seitlich öffnenden Schnittes aus der Hornhaut herausgenommen. Die Gestalt des Teilvolumens ist so gewählt, daß nach Entnahme die Brechungseigenschaften der Hornhaut so geändert sind, daß die erwünschte Fehlsichtigkeitskorrektur bewirkt ist.

Zur Isolierung des Teilvolumens ist es natürlich unumgänglich, die optischen Durchbrüche an vorbestimmten Stellen zu erzeugen. Die US 5.984.916 beschreibt entsprechende Sensoren, die Querschnitt sowie Lage und Intensität des Behandlungs-Laserstrahls erfassen und eine entsprechende Steuereinrichtung speisen, die den Laserbehandlungsstrahl so beeinflußt, daß am gewünschten Zielpunkt ein gewünschter optischer Durchbruch erreicht wird.

Die EP 1 232 734 A1 schlägt dagegen vor, einen Wellenfrontsensor zu verwenden, um die Lage eines optischen Durchbruches, der in einem Auge erzeugt wurde, zu bestimmten. In der Veröffentlichung ist diesbezüglich davon die Rede, daß die Blasengröße mit dem Wellenfrontsensor unter Verwendung "relativ gut bekannter Wellenfronttechniken" gemessen werden kann. Über die Art und Weise wie die Messung erfolgen könnte, schweigt sich die Schrift leider aus. Sie vermittelt keine technische Lehre, sondern bleibt aufgabenhaft. Da ein Wellenfrontsensor aber bekanntermaßen in der Lage ist, eine Verzerrung einer Wellenfront festzustellen, wäre es denkbar, die in EP 1 232 734 A1 aufgabenhaft genannte Zielsetzung dadurch zu erreichen, daß mit einem Wellenfrontsensor eine Verformung der Hornhautoberfläche detektiert wird, die von einer im Inneren der Hornhaut erzeugten Blase hervorgerufen wurde. Bei einem solchen denkbaren Ansatz wäre es jedoch zu erwarten, daß die Meßgenauigkeit mit abnehmender Blasengröße stark sinkt, da eine kleinere Blase natürlich auch zu einer stark verminderten Verformung der Hornhautvorderfläche führen wird. Darüber hinaus wäre zu erwarten, daß bei einer tiefer gelegenen Blase der durch diese Art der Messung angezeigte Blasendurchmesser geringer ausfällt, als bei einer höher gelegenen Blase gleicher Größe. Mit einem solchen Ansatz müßte also mit einem von der Tiefenlage der Blase abhängigen Meßfehler gerechnet werden.

Die DE 19635998 C, WO 01/91661 A und WO 01/80792 A befassen sich mit der Koagulation absorbierender Augenstrukturen. Die Juhasz T. el al: "Time-Resolved Observations of Shock Waves and Cavitation Bubbles Generated by Femtosecond Laser Pulses in Corneal Tissue and Water", Lasers in Surgery and Medicine, Wiley-Liss, New York, US, Bd. 19, Nr. 1, 1996, Seiten 23-31, XP000597102, ISSN: 0196-8092, verwendet Blitzlichtfotografie in einem Experiment Rinder-Augenhornhaut, die zwischen zwei Glasplatten eingeklemmt ist, in Durchlichtabbildung während der Erzeugung optischer Durchbrüche abzubilden.

Die Genauigkeit, mit der Behandlungs-Laserstrahlung z.B. zur Fehlsichtigkeitskorrektur am Auge eines Patienten appliziert werden kann, hat natürlich direkte Auswirkung auf die Qualität des Ergebnisses, im genannten Beispiel auf die Qualität einer optischen Korrektur. Es ist deshalb Aufgabe der Erfindung, eine Vorrichtung zur Messung eines optischen Durchbruches zu schaffen, mit der eine gesteigerte Zielgenauigkeit der Wirkung einer Behandlungslaserstrahlung erreicht werden kann.

Diese Aufgabe wird mit einer Vorrichtung gemäß Anspruch 1 gelöst. Der optische Durchbruch wird in einem Gewebe unterhalb einer Gewebeoberfläche von der Behandlungs-Laserstrahlung ausgelöst, die die laser-chirurgische Einrichtung in einem im Gewebe liegenden Behandlungsfokus bündelt, wobei die Vorrichtung den Detektionsstrahlengang mit der Optik aufweist. Die Optik koppelt aus dem Gewebe unterhalb der Gewebeoberfläche ausgehende Strahlung in den Detektionsstrahlengang ein, und dem Detektionsstrahlengang ist eine Detektoreinrichtung nachgeordnet, die ein Detektionssignal erzeugt, das räumliche Ausdehnung und/oder Lage des optischen Durchbruchs im Gewebe anzeigt.

Im erfindungsgemäßen Konzept wird also die räumliche Ausdehnung und/oder Lage des optischen Durchbruches detektiert und dazu Strahlung analysiert, die aus dem Gewebe selbst, d.h. unter der Gewebeoberfläche ausgeht, wobei unter dem Begriff "ausgeht" rückgestreute, reflektierte oder induzierte Strahlung im Gegensatz zu transmittierter Strahlung zusammengefaßt ist. Die erfindungsgemäße Vorrichtung eignet sich für die Vermessung bei Behandlungen von transparentem Gewebe, da in diesem Fall bis zu dreidimensionale Strukturinformationen gewonnen werden können. Damit ist die Erfindung besonders für die Überwachung mikrochirurgischer Operationen am Auge geeignet.

Das Detektionssignal kann zur Steuerung der Behandlungsstrahlung auf vielfältige Art und Weise verwendet werden. So kann zum einen ein die Ausdehnung eines optischen Durchbruches anzeigendes Detektionssignal zur Steuerung von Laserparametern, z. B. Strahlquerschnitt, Strahlungsintensität und/oder Pulsdauer eingesetzt werden. Dabei ist eine manuelle Einstellung durch einen Beobachter, dem das Detektionssignal dargeboten wird, ebenso denkbar, wie eine teilautomatische oder vollautomatische Regelung. Die Beeinflussung der Behandlungs-Laserstrahlung kann dabei Parameter beeinflussen, die den Strahl formen, wie auch Ablenkungsparameter, die den Strahl im Gewebe örtlich steuern. Bei einer Erfassung der räumlichen Ausdehnung des optischen Durchbruches kommt vordringlich eine Beeinflussung der die Strahleigenschaften steuernden Parameter in Frage, wogegen ein die Lage des optischen Durchbruchs im Gewebe anzeigendes Detektionssignal besonders für die Steuerung einer Ablenkeinrichtung geeignet ist und somit die Führung des Laserstrahls mittels einer Regelschleife ermöglicht.

Das von der Detektoreinrichtung erzeugte Detektionssignal kann vorzugsweise direkt zur Regelung der Behandlungs-Laserstrahlung verwendet werden. Dabei ist die Regelschleife unter Berücksichtigung des tatsächlich im Gewebe entstehenden bzw. wirkenden optischen Durchbruches geschlossen. Dies ist unmittelbarer, als die Verwendung von vor der Wechselwirkung mit dem Gewebe abgegriffenen Zwischenwerten. Durch Detektion der Strahlung aus dem Gewebe ist eine sehr viel genauere Regelung der Behandlungs-Laserstrahlung möglich, da der optische Durchbruch direkt erfaßt wird. Die im Stand der Technik bisher vorgeschlagene Abfühlung der Behandlungs-Laserstrahlungsparameter vor dem Eindringen in das Gewebe ist sehr viel indirekter, da die Wechselwirkung mit dem Gewebe unberücksichtigt bleibt, wohingegen der erfindungsgemäße Ansatz eine unmittelbare Regelung leistet.

Besonders bevorzugt für die Behandlungs-Laserstrahlung sind ultra-kurze Laserpulse mit Impulsdauern zwischen 1 fs und 100 ps im infraroten oder sichtbaren Spektralbereich (400 bis 1900 nm). Diese optische Strahlung kann Gewebe, insbesondere die transparenten Teile des Auges (Hornhaut, Linse, Glaskörper) durchdringen. Bei hohen Leistungsdichten, die nur im Fokuspunkt erreicht werden, lösen die Laserpulse nicht-lineare, optische Prozesse in Form von optischen Durchbrüchen aus, wie z.B. durch Mehrphotonenanregung oder Frequenzkonversion bedingt, wobei die erforderliche Leistungsdichte gewebespezifisch sein kann.

Die Erfindung ist insbesondere für das erwähnte zur Fehlsichtigkeitskorrektur vorgesehene Operationsverfahren geeignet. Daneben kann die Erfindung auch für andere ophtamologische oder sonstige chirurgische Eingriffe verwendet werden. Dazu gehören Schnitte für die refraktive Augenchirurgie oder zur Entfernung von eingeschlossenen Fremdkörpern, Schnitte in der Hornhaut, Schnitte im Glaskörper des Auges, in der Linse oder der Sklera. Ebenso sind lokalisierte laserinduzierte Gewebeveränderungen ohne Schnittwirkungen denkbar, die Trübungen oder Verhärtungen der Hornhaut verringern. Aber auch andere Gewebe sind für Infrarotstrahlung transparent, beispielsweise die Dermis. Bei entsprechender Wahl der Wellenlänge der Behandlungs-Laserstrahlung sind auch in diesen Geweben Diagnosen und Behandlungen möglich. Auch für eine in vitro Gewebebearbeitung ist die Erfindung geeignet. So können beispielsweise in einem extrahierten Gewebestück histologische Untersuchungen durchgeführt werden. Der Behandlungs-Laserstrahl kann zur Ausführung eines histologischen Schnittes in eine gegebenenfalls zuvor vermessene Geweberegion benutzt werden. Die Schnitteigenschaften der ultrakurzen Laserpulse wirken sich dabei vorteilhaft auf die Qualität der Schnittfläche auf; kollaterale Gewebeschäden sind weitgehend ausgeschlossen.

Jeder optische Durchbruch wirkt sich auf das Gewebe aus. In den meisten Fällen bildet sich zumindest kurzzeitig ein optisches Streuzentrum, z.B. in Form einer Plasmablase. Es ist deshalb bevorzugt, daß die räumliche Ausdehnung und/oder Lage von durch den optischen Durchbruch erzeugten Streuzentren bestimmt wird.

Die aus dem Gewebe ausgehende Strahlung kann in einer besonders vorteilhaften Ausgestaltung der Erfindung rückgestreute Strahlung sein, die aus einer Beleuchtungsstrahlungsquelle stammt. Es ist deshalb in der Vorrichtung eine Beleuchtungsstrahlungsquelle, die Beleuchtungsstrahlung in das Gewebe einkoppelt, vorgesehen. Beleuchtungsstrahlung und Behandlungsstrahlung können aus ein- und derselben Strahlungsquelle abgeleitet werden, z.B. durch Einsatz eines einschaltbaren Energieminderers. Alternativ zu extern eingekoppelter Beleuchtung können auch rückgestreute Anteile der Behandlungs-Laserstrahlung für die Detektion verwendet werden.

Eine für die Führung des Laserstrahls bei der erwähnten Operationsmethode geeignete Detektion der Lage des optischen Durchbruchs erfordert eine Genauigkeit, die durch eine gewünschte Fehlsichtigkeitskorrektur vorgegeben ist. Je nach optischer Anwendung kann ein Dickenunterschied zwischen Mitte und Rand eines zu isolierenden Volumens von etwa 12 µm eine Brechkraftveränderung der Hornhaut von einer Dioptrie bewirken. Eine entsprechend exakte räumliche Vermessung der Lage des optischen Durchbruchs ist deshalb insbesondere für ophtalmologische Operationsanwendungen von großem Vorteil.

In der Erfindung findet eine interferometrische Detektion der aus dem Gewebe ausgehenden Strahlung statt, und die Vorrichtung weist eine Beleuchtungsstrahlungsquelle auf, die zusammen mit dem Detektionsstrahlengang Teil eines Interferometeraufbaus ist. Zweckmäßigerweise wird rückgestreute Strahlung detektiert und die Information über den optischen Durchbruch, der von der Behandlungs-Laserstrahlung im Gewebe erzeugt wird, insbesondere die Lokalisierung des optischen Durchbruchs in Bezug auf angrenzende Gewebeschichten, wird aus dem vom Ort des Fokus der Beleuchtungsstrahlung rückgestreuten Strahlung gewonnen. Die Menge an rückgestreuter Strahlung trägt Informationen über Brechzahlsprünge, die sowohl an der Grenzschicht verschiedener Gewebearten (z.B. zwischen Stroma und Bowman'scher Membran einer Hornhaut) als auch am Ort des optischen Durchbruchs sowohl unterhalb als auch oberhalb der Schwelle zur Photodisruption und - ablation auftreten.

Ein solcher Interferometeraufbau ist als optischer Kohärenztomograph ausgebildet, der aus dem Gewebe ausgehende Strahlung, z.B. rückreflektierte oder gestreute Beleuchtungsstrahlung, in geeigneter Form räumlich filtert. Die räumliche Filterung erfolgt dabei derart, daß der optische Durchbruch mit ausreichender Genauigkeit lokalisiert wird. Wird die Beleuchtungsstrahlung entlang einer optischen Achse eingestrahlt, kann die räumliche Filterung lateral, d.h. senkrecht zur optischen Achse durch eine geeignete Fokussierung der Beleuchtungsstrahlung erreicht werden. Die Fokuslage der Beleuchtungsstrahlung legt dann in lateraler Richtung das Meßvolumen fest, in dem ein optischer Durchbruch detektiert bzw. vermessen wird. In axialer Richtung kann bei einem optischen Kohärenztomographen die Lokalisierung des Meßvolumens durch eine kurze zeitliche Kohärenz der Beleuchtungsstrahlung erreicht werden. Interferenz tritt dann nur bei Rückstreuung aus dem Meßvolumen auf, und das Vorliegen einer Interferenz zeigt dann, daß Strahlung aus einer bekannten Tiefe innerhalb des Gewebes rückgestreut wurde. Da der optische Durchbruch, wie bereits erwähnt, mit dem Entstehen einer Photodisruptions- oder Plasmablase einhergeht und Gewebeschichten trennt, ist der optische Durchbruch durch eine lokal signifikant gesteigerte Rückstreuung von Beleuchtungsstrahlung gekennzeichnet. Es wird also bevorzugt aus dem Auftreten einer Interferenz die Lage der Rückstreuung der aus dem Gebiet des optischen Durchbruches ausgehenden Strahlung auf der optischen Achse der Detektion bestimmt.

Es ist diesbezüglich bevorzugt, daß der Interferometeraufbau einen Meßarm und einen verstellbaren Referenzarm aufweist. Die Kohärenzlänge der Beleuchtungsstrahlung bestimmt die Auflösung in axialer Richtung, da Interferenz nur auftritt, wenn Länge von Meßarm und Referenzarm sich um weniger als die Kohärenzlänge der Beleuchtungsstrahlung unterscheiden. In Kombination mit der lateralen Diskriminierung durch die Fokussierung der Beleuchtungsstrahlung ist in summa eine dreidimensionale örtliche Detektion des optischen Durchbruches bewirkt mit dem Durchmesser der Fokussierung die laterale Auflösung und der Kohärenzlänge der Beleuchtungsstrahlung die Tiefenauflösung festlegend.

Um nun ein größeres Gebiet auf optische Durchbrüche abzutasten, ist es zweckmäßig, daß die Beleuchtungsstrahlungsquelle die Beleuchtungsstrahlung in einem im Gewebe gelegenen Beleuchtungsfokus bündelt, dessen Lage zur Erzeugung des Detektionssignals verstellbar ist. Die Verstellung erfolgt dabei vorteilhafterweise bezogen auf die Lage des Fokusses der Behandlungs-Laserstrahlung. Um ein möglichst einfach aufgebautes und kompaktes Gerät zu erreichen, ist es zu bevorzugen, daß die Behandlungs-Laserstrahlung und die Beleuchtungsstrahlung von derselben Optik auf das zu behandelnde Gewebe, beispielsweise auf die Hornhaut eines Auges, gerichtet werden. Dies wird man in der Regel dadurch erreichen, daß Beleuchtungsstrahlung und Behandlungs-Laserstrahlung über eine optische Koppeleinheit zusammengeführt werden, beispielsweise über einen Strahlteiler. Die dann von beiden Strahlen gemeinsam passierte Fokussieroptik erzeugt dann einen Fokus, der lateral und je nach Strahldivergenz vor der Zusammenführung auch axial etwa am selben Ort liegt.

Eine eigenständige laterale Verschiebung der Beleuchtungsstrahlung gegenüber der Behandlungs-Laserstrahlung kann erreicht werden, wenn vor der Zusammenführung eine verstellbare Ablenkeinrichtung, beispielsweise ein Scanner vorgesehen ist. Die Fokuslage der Beleuchtungsstrahlung kann in axialer Richtung durch Anpassung der Divergenz der Beleuchtungsstrahlung vor der Zusammenführung mit der Behandlungs-Laserstrahlung verstellt werden. Analog kann durch geeignete Vorkonditionierung des Durchmessers des Strahls der Beleuchtungsstrahlung die Fokusgröße gegenüber der Behandlungs-Laserstrahlung verändert werden.

Es ist deshalb diesbezüglich bevorzugt, daß die Beleuchtungsstrahlung in einen Lichtweg der Behandlungs-Laserstrahlung eingekoppelt ist, wobei eine verstellbare Optik verwendet ist, mit der die Divergenz der Beleuchtungsstrahlung ohne Veränderung der Divergenz der Behandlungs-Laserstrahlung veränderbar ist.

Die Beobachtung des optischen Durchbruchs erfolgt beim optischen Kohärenztomographen bevorzugt durch eine axiale Verstellung der räumlichen Selektion der rückgestreuten Strahlung unter Einschluß des Ortes des optischen Durchbruchs. Dadurch kann sowohl die axiale Ausdehnung des optischen Durchbruchs als auch dessen Lage beobachtet werden und das Detektionssignal gewonnen werden. Die beim optischen Kohärenztomographen erfolgende Heterodyndetektion der rückgestreuten Strahlung verwendet vorzugsweise eine Beleuchtungsstrahlung mit möglichst großer Bandbreite, bevorzugt um eine mittlere Wellenlänge von 850 nm. Die Bandbreite ist umgekehrt proportional zur Kohärenzlänge der Beobachtungsstrahlung, die in der Heterodyndetektion die axiale Auflösung des optischen Kohärenztomographen mitbestimmt. Durch die Verwendung verschiedener Wellenlängen von Beleuchtungsstrahlung und Behandlungs-Laserstrahlung kann eine Überlagerung und Trennung der beiden Strahlengänge mit dichroitischen Strahlteilern einfach erfolgen.

Für die Realisierung der erwähnten axialen Verschiebung des Fokusses gibt es prinzipiell zwei Möglichkeiten:
a) Ist der Fokusdurchmesser der Beleuchtungsstrahlung größer als der des Behandlungs-Laserstrahls, kann ein Abtasten durch ein geeignetes Verstimmen des Interferometer, z. B. durch Verstellen des Meßarmes erreicht werden. Die axiale Auflösung ist dann hauptsächlich durch die Kohärenzlänge der Beleuchtungsstrahlung bestimmt und liegt typischerweise in der Größenordnung von 10 µm. Der Verstimmbereich legt den Meßbereich axial fest.
b) Ist der Fokusdurchmesser der Beleuchtungsstrahlung und der Behandlungs-Laserstrahlung in ähnlicher Größe, setzt ein axiales Abtasten eine synchrone Verstimmung des Interferometers und der Fokuslage voraus. Letzteres kann mittels der verstellbaren Optik, mit der die Diverengenz der Beleuchtungsstrahlung ohne Veränderung der Divergenz der Behandlungs-Laserstrahlung veränderbar ist, vor der Zusammenführung von Beleuchtungsstrahlung und Behandlungs-Laserstrahlung erfolgen. Die axiale Auflösung der Detektion des optischen Durchbruchs ist dann sowohl von der Tiefenschärfe der durch die Heterodyndetektion inhärent konfokalen optischen Abbildung als auch von der Kohärenzlänge der Beleuchtungsstrahlung abhängig und liegt unter der erwähnten Auflösung für Fall a).

Die im Fall b) erreichte höhere Auflösung kommt natürlich auch einer lateralen Abtastung des Gebietes, in dem der optische Durchbruch erwartet bzw. erzeugt wird, zugute. Andererseits ist dann eine erhöhte Verstellgeschwindigkeit erforderlich, um ein Gebiet bestimmter Größe in gleicher Zeit abzutasten.

In den geschilderten Ausführungsformen, in denen Detektionsstrahlengang und BeleuchtungsStrahlengang im wesentlichen auf einer gemeinsamen optischen Achse einfallen, ist zwar ein sehr kompakt bauendes Gerät erreichbar, jedoch kann mitunter eine an und für sich wünschenswerte Information über Strukturen, die auf der optischen Achse hinter dem optischen Durchbruch liegen, nur sehr schwer erhalten werden, da der optische Durchbruch für Strahlung aus dahinterliegenden Gewebeabschnitten meist eine abschattende Wirkung hat. Für Anwendungen, in denen auch eine optische Detektion in Gewebebereichen erfolgen sollen, die von der Vorrichtung aus gesehen hinter dem optischen Durchbruch liegen, ist es deshalb zweckmäßig, daß der Detektionsstrahlengang eine optische Achse aufweist, die schräg zu einer optischen Achse der Behandlungs-Laserstrahlung oder einer Beleuchtungsstrahlung liegt. Die ausgehende Strahlung wird also entlang einer optischen Achse detektiert, welche schräg zu einer optischen Achse liegt, entlang der die Behandlungs-Laserstrahlung oder eine Beobachtungsstrahlung in das Gewebe eingestrahlt wird. Dieser Ansatz erlaubt es beispielsweise die Dicke einer Hornhaut gleichzeitig mit der Erfassung des optischen Durchbruches zu bestimmen. Die Dickenbestimmung ermöglicht wiederum eine einfache Skalierung der Abmessung des optischen Durchbruches, beispielsweise eines Plasmablasendurchmessers da die Hornhautdicke ein gut bekanntes und insbesondere vor ophtalmologischen Eingriffen in der Regel exakt vermessenes Maß liefert. Damit ist auch zusätzlich eine automatische Kalibrierung zur Vermessung des optischen Durchbruches erreicht.

Eine solche Scanvorrichtung ist für die erfindungsgemäße Vorrichtung allgemein nützlich und immer dann vorteilhaft, wenn ein um einen zu erwartenden optischen Durchbruch liegendes Gebiet abgescannt werden soll. Es ist deshalb bevorzugt, daß eine Scanvorrichtung zum Abscannen des Gewebes verwendet wird.

Die Erfindung wird nachfolgend unter Bezugnahme auf die Zeichnung beispielshalber noch näher erläutert. In den Zeichnungen zeigen:
- Fig. 1: eine perspektivische Darstellung eines Patienten während einer laserchirurgischen Behandlung mit einem laserchirurgischen Instrument,
- Fig. 2: eine Schemadarstellung des laserchirurgischen Instrumentes der Fig. 1,
- Fig. 3: die Fokussierung eines Strahlenbündels auf das Auge des Patienten beim Instrument der Fig. 2,
- Fig. 4: einen Signalverlauf bei der Vermessung von Streuzentren, die während der laserchirurgischen Behandlung im Auge erzeugt werden,
- Fig. 5: eine schematische Darstellung zur Erläuterung einer während der laserchirurgischen Behandlung ausgeübten Schnittführung,
- Fig. 6: eine Lichtquelle für das laserchirurgische Instrument der Fig. 2,
- Fig. 7: eine Scanvorrichtung für das laserchirurgische Instrument der Fig. 1 und
- Fig. 8: eine weitere Ausführungsform einer Meßvorrichtung des laserchirurgischen Instruments der Fig. 1.

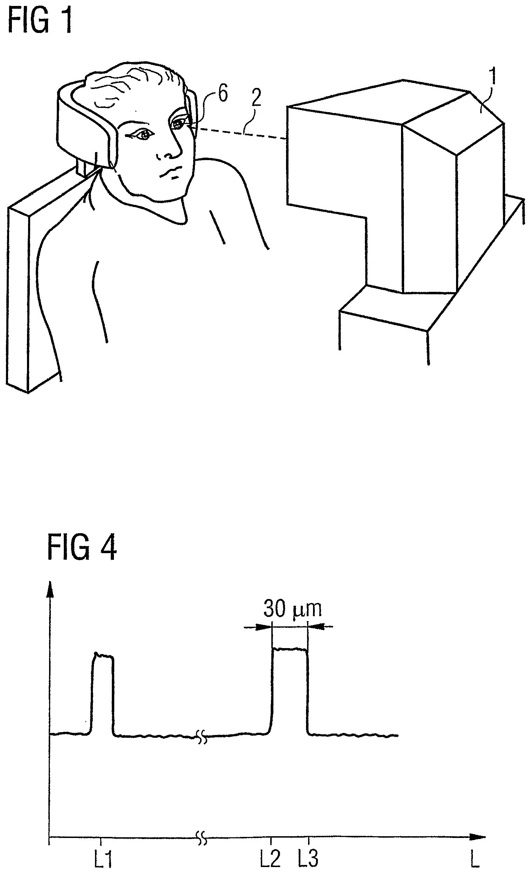

In Fig. 1 ist ein laserchirurgisches Instrument 1 dargestellt, das einen Behandlungsstrahl 2 abgibt, welcher auf das Auge 6 eines Patienten gerichtet ist. Das laserchirurgische Instrument 1 ist dabei in der Lage, einen gepulsten Behandlungsstrahl 2 zu erzeugen, so daß das in US 6.110.166 beschriebene Verfahren ausgeführt werden kann. Die Pulsdauer liegt dabei im Nano-oder Femtosekundenbereich.

Mittels des laserchirurgischen Instrumentes 1 wird eine Fehlsichtigkeit des Auges 6 des Patienten dadurch behoben, indem aus der Hornhaut des Auges 6 Material so entfernt wird, daß sich die Brechungseigenschaft der Hornhaut um ein gewünschtes Maß ändert. Das Material wird dabei dem Stroma der Hornhaut entnommen, das unterhalb von Epithel und Bowmanscher Membran und oberhalb der Decemetscher Membran und des Endothels liegt.

Die Materialentfernung erfolgt, indem durch Fokussierung des hochenergetischen gepulsten Behandlungsstrahls 2 des laserchirurgischen Instrumentes 1 im Stroma Gewebeschichten getrennt werden. Jeder einzelne optische Durchbruch initiiert dabei eine Plasmablase, so daß die Gewebeschichttrennung ein größeres Gebiet erfaßt, als der Fokus des Behandlungsstrahls 2. Durch geeignete Ablenkung des Behandlungsstrahls 2 werden während der Behandlung Plasmablasen aneinandergereiht. Die aneinanderliegenden Plasmablasen umschreiben dann ein Teilvolumen des Stromas: das zu entfernende Material.

Das laserchirurgische Instrument 1 wirkt durch den Behandlungsstrahl 2 wie ein chirurgisches Messer, das, ohne die Oberfläche der Hornhaut zu verletzten, direkt Material im Inneren des Stromas schneidet. Führt man den Schnitt durch weitere Erzeugung von Plasmablasen bis an die Oberfläche der Hornhaut, kann das durch die Schnittführung isolierte Material des Stromas seitlich aus der Hornhaut herausgezogen und somit entfernt werden.

Die Schnittführung des laserchirurgischen Instrumentes 1 wird gemäß zuvor ermittelten Parametern durchgeführt, damit das entfernte Teilvolumen des Stromas eine derartige Veränderung der optischen Eigenschaften der Hornhaut bewirkt, daß eine zuvor bestehende Fehlsichtigkeit weitestmöglich korrigiert ist.

Zur Überwachung der genauen Schnittführung ist im laserchirurgischen Instrument 1 eine Meßvorrichtung vorgesehen, die die räumliche Ausdehnung und/oder die Lage der vom Behandlungsstrahl 2 im Hornhautgewebe des Auges erzeugten Plasmablasen erfaßt. Die Messung erlaubt es, den Behandlungsstrahl 2 optimal zu steuern. Die Steuerung kann dabei sowohl die scannende Bewegung des Behandlungsstrahls 2, als auch die Steuerung der Strahlparameter des Behandlungsstrahls 2 in Bezug auf die Erzeugung des optischen Durchbruches betreffen. Der Durchmesser der erzeugten Plasmablasen ist für die Führung des Laserstrahls beim Scanvorgang von großer Bedeutung, da der Abstand zwischen einzelnen Plasmablasenzentren den mittleren Plasmablasendurchmesser nicht überschreiten sollte. Ansonsten wäre eine Abfolge von Plasmablasen lückenhaft, d.h. es verbliebe zwischen den disruptierten Volumina immer noch zusammenhängendes Gewebe. Eine Isolierung eines zu entfernenden Stromamaterials wäre dann nur schwer oder sogar gar nicht möglich. Auf jeden Fall wäre das optische Resultat unbefriedigend.

Die Optimierung der Parameter des Behandlungsstrahls 2 erlaubt es aber auch die Plasmablasen so klein wie möglich zu halten. Je kleiner die Plasmablasen, desto feiner ist der vom laserchirurgischen Instrument 1 erzeugte Schnitt. Dies ist insbesondere dann wichtig, wenn man berücksichtigt, daß in der Regel linsenförmige Teilvolumina aus dem Stroma entfernt werden sollen. Am Rande dieser linsenförmigen Teilvolumina sind die Präzision der Schnittführung und die Feinheit des Schnittes besonders wichtig.

Die Meßwerte der Meßvorrichtung können entweder in eine Anzeige am laserchirurgischen Instrument 1 umgesetzt werden, die es erlaubt, die Parameter des Behandlungsstrahls 2 bzw. die Führung des Behandlungsstrahls 2 daraufhin zu überprüfen, ob gewünschte und festgelegte Werte auch tatsächlich vorliegen. Alternativ ist es möglich, mittels einer automatischen Prozeßkontrolle eine Regelung der Parameter des Behandlungsstrahls 2 bzw. der Ablenkung des Behandlungsstrahls 2 auf bestimmte Werte hin vorzunehmen. Das laserchirurgische Instrument 1 arbeitet dann vollautomatisch.

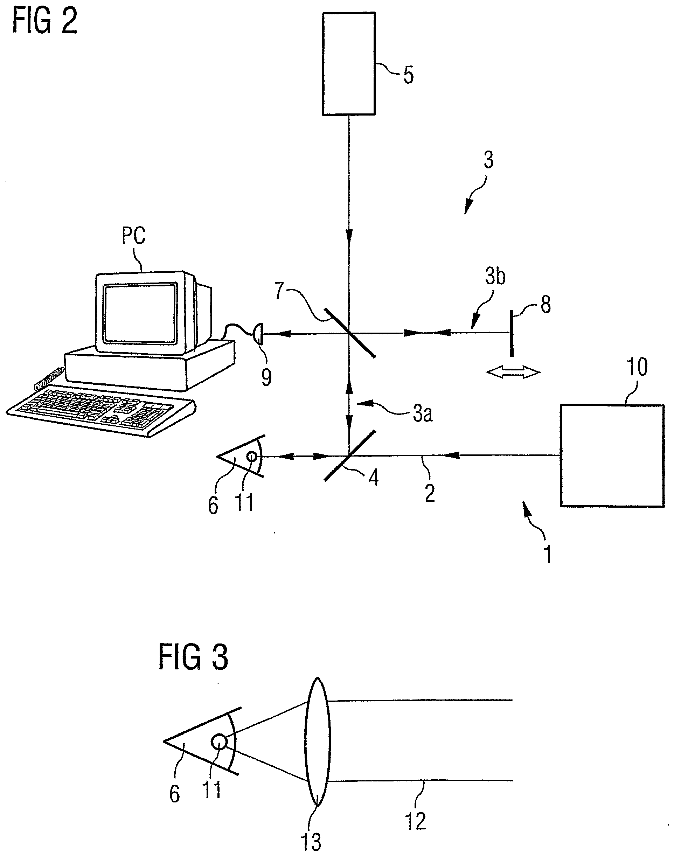

Fig. 2 zeigt eine Ausführungsform des laserchirurgischen Instruments 1. Die Meßvorrichtung ist hier als optischer Kohärenztomograph 3 aufgebaut und folgt dem Prinzip, wie es in der Veröffentlichung Arch. Ophtalmol., Vol. 112, S. 1584, "Micrometer-scale Resolution Imaging of the Interior Eye in vivo with Optical Coherenztomographie" von J. Izatt et al., beschrieben ist.

Der optische Kohärenztomograph 3 ist über einen Einkoppelstrahlteiler 4 in den Behandlungsstrahl 2 des laserchirurgischen Instrumentes 1 eingebunden und weist einen Meßarm 3a sowie einen Referenzarm 3b auf. Der Einkoppelstrahlteiler 4 koppelt Strahlung eines Meßlasers 5 in den Lichtweg des laserchirurgischen Instrumentes 1 so ein, daß die optischen Achsen von Behandlungsstrahl und Meßlaserstrahl zusammenfallen. Der Fokus des Behandlungsstrahls 2 und der Fokus der Strahlung des Meßlasers 5, die beide durch eine dem Einkoppelstrahlteiler 4 nachgeordnete Fokussieroptik 13 in der Hornhaut des Auges 6 gebildet werden, befinden sich lateral etwa an derselben Stelle (in Fig. 2 nicht zu sehen).

Fig. 3 zeigt wie die bereits vereinigte Strahlung des Meßlasers 5 und des Behandlungsstrahls 2 als einfallender Strahl 12 mittels der Fokussieroptik 13 in die Hornhaut des Auges 6 fokussiert wird. Wenn die Energiedichte des Behandlungsstrahls 2 (Pulslänge und Strahlungsleistung) oberhalb eines bestimmten Schwellwertes liegt, tritt im Fokus ein optischer Durchbruch auf, der eine Plasmablase 11 erzeugt.

Die Strahlung des Meßlasers 5 wird an der Plasmablase 11 zurückgestreut, am dichroitischen Einkoppelstrahlteiler 4 wieder ausgekoppelt und gelangt dann zu einem Meßstrahlteiler 7, wo sie mit Strahlung des Meßlasers 5 überlagert wird, die zuvor vom Meßstrahlteiler 7 zu einem Spiegel 8 abgeteilt wurde. Die Lage des Spiegels 8 ist verstellbar. Der Meßarm 3a verläuft somit vom Meßstrahlteiler 7 über den Einkoppelstrahlteiler 4 zum Auge 6 und der Referenzarm 3b ist zwischen Meßstrahlteiler 7 und verstellbarem Spiegel 8 gebildet.

Die aus Referenzarm 3b und Meßarm 3a zum Meßstrahlteiler 7 zurückkehrende Strahlung wird auf einem Photoempfänger 9 zur Interferenz gebracht. Da als Meßlaser 5 eine Lichtquelle verwendet wird, die bei hoher räumlicher Kohärenz eine vergleichsweise kurze Kohärenzlänge in axialer Richtung (zeitliche Kohärenzlänge) von etwa 10 µm aufweist, erscheint an der Photodiode 9 nur dann ein Interferenzmuster, wenn die optischen Längen von Meßarm 3a und Referenzarm 3b maximal um die zeitliche Kohärenzlänge der Strahlung des Meßlasers 5 differieren. Um mit dem optischen Kohärenztomographen 3 eine möglichst hohe Auflösung zu erreichen, wird ein Meßlaser 5 mit einer möglichst kurzen zeitlichen Kohärenzlänge verwendet, im Ausführungsbeispiel liegt diese unter 10 µm.

Um eine Beeinflussung des optischen Kohärenztomographen durch den Behandlungsstrahl 2 des laserchirurgischen Instruments 1 zu verhindern, der von einem Behandlungslaser 10 abgegeben und auf das Auge 6 gelenkt wird, ist der Wellenlängenbereich der Strahlung des Meßlasers 5 vom Wellenlängenbereich der Beobachtungsstrahlung 2 verschieden und der Einkoppelstrahlteiler 4 hat geeignete dichroitische Eigenschaften. Beispielsweise wird ein Meßlaser 5 verwendet, der Strahlung bei etwa 850 nm mit einer möglichst großen Bandbreite abgibt. Eine große Bandbreite fördert die axiale Auflösung, da die Bandbreite umgekehrt proportional zur zeitlichen Kohärenzlänge des Lichtes ist. Bei Verwendung verschiedener Wellenlängen für die Strahlung des Meßlasers 5 und Beobachtungsstrahl 2 kann eine Überlagerung und Trennung der Strahlen mit einem dichroitischen Einkoppelstrahlteiler 4 erfolgen. Zusätzlich oder alternativ können im Strahlengang des optischen Kohärenztomographen geeignete Filter eingesetzt werden.

Zur Messung des Ortes einer Plasmablase 11 wird der Spiegel 8 verschoben, bis an der Photodiode 9 ein Interferenzmuster auftritt. Zeigt das Signal der Photodiode 9, das von einem Computer PC ausgelesen wird, eine Interferenz der Strahlung aus Meßarm 3a und Referenzarm 3b an, so sind Meßarm 3a und Referenzarm 3b gleich lang und der Abstand zum Fokus der Strahlung des Beobachtungslasers 5 in der Hornhaut des Auges 11 ist bekannt, wobei die Meßungenauigkeit die zeitliche Kohärenzlänge der Strahlung des Meßlasers 5 ist, d.h. die Kohärenzlänge in Strahlrichtung.

Die Rückstreuung von Strahlung des Meßlasers 5 am Auge 6 gibt eine Aussage über die Brechzahlsprünge in der Hornhaut des Auges 6, die sowohl an der Grenzschicht verschiedener Gewebe, z.B. zwischen Stroma und Bowmanscher Membran, als auch am Ort der Wechselwirkung von Behandlungsstrahl 2 und Hornhaut des Auges 6 sowohl unterhalb als auch oberhalb einer Schwelle auftreten, bei der es zum erwähnten optischen Durchbruch mit Photodisruption und/oder -ablation kommt.

Damit ein möglichst großer axialer Bereich vermessen werden kann, ist zusätzlich zu einem großen Verstellweg für den Spiegel 8 zwischen den Meßstrahlteiler 7 und den Einkoppelstrahlteiler 4 eine Einrichtung zur Veränderung der Divergenz der Strahlung des Meßlasers 5 geschaltet (in Fig. 2 zur Vereinfachung nicht gezeigt), so daß vor der Einkopplung in den Behandlungsstrahl 2 die Divergenz der Strahlung des Meßlasers 5 verstellt werden kann. Durch die dem Einkoppelstrahlteiler 4 nachgeschaltete Fokussieroptik 13 erfolgt dann die Fokussierung von Meßstrahl 2 und Strahlung des Meßlasers 5 in unterschiedliche Foki, wobei durch die Verstellung der die Divergenz der Strahlung des Meßlasers 5 beeinflussenden Einrichtung die Fokuslage der Strahlung des Meßlasers 5 unabhängig vom Fokus des Behandlungsstrahls 2 verstellt werden kann. Der Fokus der Beobachtungsstrahlung ist somit entlang der optischen Achse gegenüber dem Fokus der Beobachtungsstrahlung 2 verstellbar. Damit wird rückgestreute Strahlung auch entlang der optischen Achse des Behandlungsstrahls 2 in einem Bereich erfaßbar, der größer als der Verstellweg des Spiegels 8 ist.

Die Einrichtung zur Verstellung der Divergenz des Meßlasers 5 erlaubt es weiter, die Spotgröße der fokussierten Strahlung des Meßlasers 5 in etwa so groß zu gestalten, wie die Spotgröße des fokussierten Beobachtungsstrahls, da ein Scannen in axialer Richtung, d.h. entlang der Achse des Beobachtungsstrahls 2, dann über eine synchrone Verstellung der divergenzverändernden Einrichtung und des Spiegels 8 gelingt. Durch die bei dieser Variante relativ höhere Fokussierung der Strahlung des Meßlasers 5 liefert der Kohärenztomograph 3 damit eine axiale Auflösung, die sogar besser ist, als die zeitliche Kohärenzlänge der Strahlung des Meßlasers 5. Die Meßgenauigkeit steigt folglich. Ist dies nicht erforderlich, kann auf die die Divergenz beeinflussende Vorrichtung zwischen dem Einkoppelstrahlteiler 4 und dem Meßstrahlteiler 7 verzichtet werden. Eine axiale Verschiebung des Bereiches, von dem rückgestreute Strahlung zu Interferenz am Photoempfänger führen kann, erfolgt dann ausschließlich durch Verstellung des Spiegels 8, und die axiale Auflösung wird hauptsächlich durch die zeitliche Kohärenzlänge des Meßlasers 5 bestimmt (typischerweise in der Größenordnung von 10 µm).

Fig. 4 zeigt ein Signal, wie es mit einem laserchirurgischen Instrument 1 mit einem optischen Kohärenztomographen 3 in der Bauweise der Fig. 2 erhalten wird. Hierbei ist das Signal als Funktion der Verstellung des Spiegels 8 aufgetragen, wobei in Fig. 4 der Verstellweg L des Spiegels 8 als Variable eingezeichnet ist. Das Signal ist aus der mit dem Photoempfänger 9 detektierten Interferenzerscheinung abgeleitet und wurde vom Computer PC erzeugt. Es codiert die Modulationstiefe der Interferenz und weist immer dann einen hohen Pegel auf, wenn das Signal des Photoempfängers 9 eine Interferenzerscheinung anzeigt.

Wie Fig. 4 zeigt, tritt eine erste Interferenz bei einer Verschiebung L1 auf. Diese Interferenz ist durch am Epithel der Hornhaut rückgestreute Strahlung verursacht. Mit weiterer Verschiebung des Spiegels 8 tritt ein zweiter Peak des Signals bei einer Verschiebung L2 auf. Diese Interferenz ist durch den Brechzahlsprung an der Plasmablase 11 verursacht. Die Interferenz hält bis zu einer Verschiebung L3 an. Die Größe der Plasmablase wirkt sich zwar auf den Abstand L2, L3 aus, jedoch erlaubt es in der realisierten Ausführungsform die Meßauflösung des Kohärenztomographen 3 nicht, den bei etwa 30 µm oder darunter liegenden Blasendurchmesser zu vermessen. Bei Verwendung einer Strahlungsquelle mit kürzerer zeitlicher Kohärenzlänge ist auch eine solche Durchmesservermessung möglich.

Der Abstand zwischen der dem Epithel zugeordneten Verschiebung L1 und dem Beginn des Interferenzpeaks bei der Verschiebung L2 gibt an, wie tief die Plasmablase unter dem Epithel, d.h. der Hornhautoberfläche liegt. Aus dem Signal kann so die Lage der Plasmablase 11, die vom Behandlungsstrahl 2 in der Hornhaut des Auges 6 verursacht wurde, in Form des Abstandes vom Epithel erfaßt werden. Alternativ zum Bezug auf das Epithel kann natürlich eine Referenzierung auf das Endothel erfolgen. Hier ist in der Darstellung der Fig. 4 bei einer weitergehenden Verschiebung ein erneuter Peak durch am Endothel rückgestreute Strahlung zu erwarten. Anstelle des Endothels bzw. des Epithels kann natürlich auch ein Brechzahlsprung an der Bowmanschen oder Decementschen Membran als Bezug verwendet werden.

Der Computer PC, der das Signal des optischen Kohärenztomographen 3 ausliest, generiert das Signal. Er dient weiter zur Steuerung des Behandlungslasers 10, wozu er zum einen eine Anzeige speist, die die Lage der vom Behandlungsstrahl 2 erzeugten Plasmablase 11 angibt. Zum anderen wird diese axiale Lage der Plasmablase zur Steuerung des Behandlungsstrahls 2 ausgewertet und verwendet, um zu garantieren, daß der Schritt zur Isolierung des Teilvolumens im Stroma wie gewünscht ausgeführt wird.

In einer vollautomatisch die Einhaltung von behandelnden Chirurgen vorgegebener Werte überwachenden Ausführung regelt der Computer Parameter des Behandlungsstrahls 2. Das chirurgische Instrument arbeitet dann mit einer Online-Kontrolle und -Regelung.

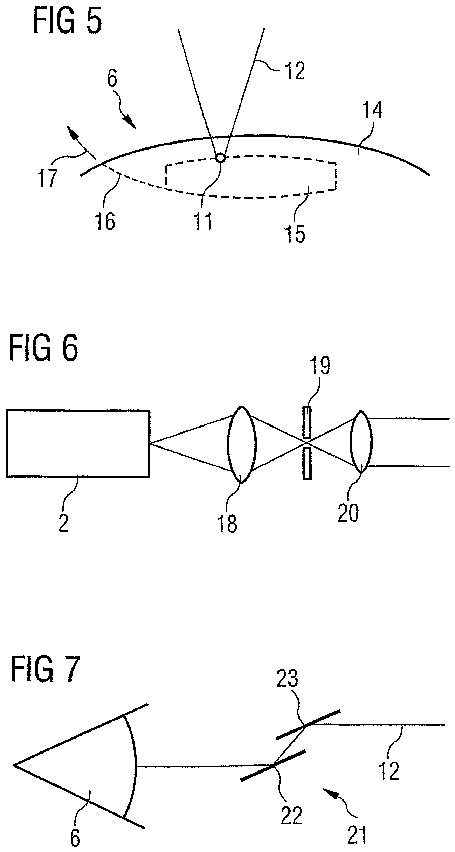

Der Meßlaser 5 ist wie in Fig. 6 gezeigt aufgebaut. Er besteht aus einem Laser 2 sowie einer nachgeordneten strahlformenden Einheit aus einer Linse 18, einer Blende 19 und einer weiteren Linse 20. Der so erzeugte Strahldurchmesser ist an den Strahldurchmesser des Behandlungsstrahls 2 angepaßt. Durch Verstellung der Linsen 18 und 20 kann zur Auflösungs-oder Meßbereichsverstellung der Strahldurchmesser zusätzlich verkleinert oder vergrößert werden. Zudem kann durch Verstellung der Linse 20 die Divergenz verändert werden und damit die Fokuslage des Meßstrahles eingestellt werden.

Fig. 5 zeigt in einer schematischen Darstellung den unter Online-Kontrolle vom chirurgischen Instrument 1 ausgeführten Schnitt. Der einfallende Strahl 12 wird von der Fokussieroptik 13 in die Hornhaut 14 des Auges 6 fokussiert. Die Plasmablase 11, die mit einem Puls des Behandlungsstrahls 2 erzeugt wird, wirkt, wie bereits ausgeführt, als chirurgisches Messer, das im Inneren der Hornhaut 14 Gewebeschichten trennt. Durch Verstellung des einfallenden Strahls 12 mittels einer in Fig. 7 dargestellten Scaneinrichtung 21 aufweisend um orthogonale Achsen drehbare Scannerspiegel 22, 23 wird eine Vielzahl von Plasmablasen nebeneinander gesetzt und ein Lenslett 15 umschrieben, das aus dem Stroma der Hornhaut 14 herausgeschnitten wird. Eine mögliche Schnittform, dies zu erreichen, ist in der US 6.110.166 dargestellt, die eine spiralförmige Aneinanderreihung von Plasmablasen 14 beschreibt.

Nach dieser Schnittführung um das Lenslett 15 herum wird ein seitlicher Schnitt 16 vorgenommen, der es erlaubt, das nunmehr isolierte Lenslett 15 in Richtung 17 aus der Hornhaut 14 zu entnehmen. Die Schnittführung hat dabei den Vorteil, daß im zentralen Sichtbereich, in dem die optische Korrektur durch Entnahme des Lensletts 15 bewirkt wird, kein durch das Endothel führender Schnitt nach außen vorliegt. Stattdessen liegt der seitliche Schnitt 16 am optisch weniger bedeutsamen Rand der Hornhaut 14.

Zum Verstellen des einfallenden Strahles 12 dient die Scaneinrichtung 21, die in Fig. 7 dargestellt ist. Sie weist zwei Scannerspiegel 22, 23 auf, die unabhängig voneinander um zwei senkrecht zueinanderliegende Achsen drehbar sind. Damit kann der einfallende Strahl 12 zweidimensional über die Hornhaut 14 geführt werden. Eine axiale Verstellung des einfallenden Strahls 12 erfolgt durch Verstellung der Fokussieroptik 13, d.h. durch Veränderung der Lage des Fokuspunktes, in dem die für einen optischen Durchbruch ausreichende Energiedichte und somit die Plasmablase 11 entsteht.

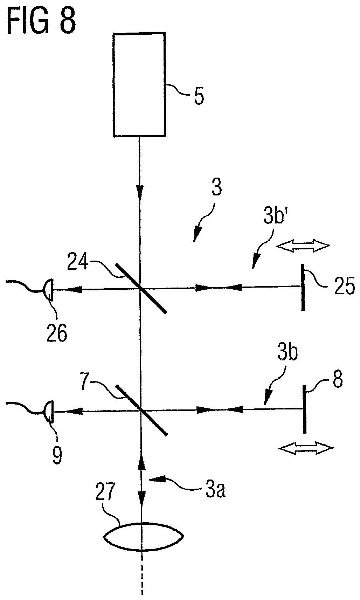

Um die axiale Lage der erzeugten Plasmablase genau zu bestimmen, wird, wie anhand des in Fig. 4 dargestellten Signals geschildert, der Spiegel 8 des optischen Kohärenztomographen 3 verstellt. Dies kann beispielsweise durch eine Oszillation erfolgen, die jedesmal den Bereich zwischen den Stellungen L1 und L2 durchläuft. Im Falle einer axial hochauflösenden Meßeinrichtung kann eine solche große Verstellung des Spiegels 8 jedoch mitunter mechanisch sehr aufwendig bzw. zeitraubend sein. Für solche Fälle ist die in Fig. 8 schematisch dargestellte Bauweise des optischen Kohärenztomographen 3 vorgesehen, die mehrere, z. B. zwei Referenzarme 3b und 3b' aufweist, welche sich einander im wesentlichen gleichen, d.h. jeweils einen verstellbaren Spiegel 8 (Referenzarm 3b) bzw. 25 (Referenzarm 3b') haben. Jedem Referenzarm 3b, 3b' ist ein Photoempfänger 9, 26 zugeordnet.

Die Länge des Referenzarmes 3b kann unabhängig von der Länge des Referenzarmes 3b' eingestellt werden. Somit kann gleichzeitig mit hoher Auflösung an der Plasmablase und am Epithel rückgestreute Strahlung, indem z.B. der Referenzarm 3b' auf den Reflex zu einer Referenzfläche, d.h. dem Epithel eingestellt wird, und der Referenzarm 3b die Plasmablasengrenzfläche erfaßt.

Fig. 8 zeigt darüber hinaus eine mögliche Bauweise der bereits bezüglich Fig. 2 angesprochenen aber dort nicht eingezeichneten Einrichtung zur Veränderung der Divergenz der Strahlung des Meßlasers 5. Beim Kohärenztomographen der Fig. 8 handelt es sich um eine verstellbare Zoom-Optik 27.

Der optische Kohärenztomograph 3, wie er in den Bauweisen gemäß Fig. 2 oder 8 zur Anwendung kommt, bewirkt eine räumliche Selektion des Gebietes, aus dem rückgestreute Strahlung aufgenommen wird. Eine axiale Selektion erfolgt durch die Interferometerkonstruktion mit dem Meßarm 3a und dem Referenzarm 3b (bzw. weiteren Paaren davon). Die Fokussieroptik 13 bewirkt eine laterale Selektion durch die Fokussierung. Die räumliche Selektion ist frei wählbar, d.h. unabhängig von der Lage des Behandlungsstrahlfokus, wenn der optische Kohärenztomograph mit einer eigenständigen Scaneinrichtung ähnlich der Fig. 7 ausgestattet ist, da dann mit hoher Auflösung das Gebiet um einen optischen Durchbruch bzw. eine Plasmablase 11 abgescannt werden kann. Mit der Zoom-Optik 27 kann das Gebiet der Plasmablase 11 nicht nur lateral, d.h. quer zur optischen Achse des Behandlungsstrahls 2, sondern auch axial entlang dieser optischen Achse in einem großen Meßbereich angefühlt werden.

Die geschilderten Ausführungsformen können besonders vorteilhaft für das eingangs erwähnte Operationsverfahren eingesetzt werden. Dazu kann man die Hornhaut des Auges an einem Kontaktglas durch Ansaugen mit hoher Tiefenauflösung (ca. 1 µm) und geringer lateraler Auflösung (z.B. 100 µm) vom Epithel bis zum Endothel über den gesamten Bereich, in dem der chirurgische Eingriff erfolgen soll, vermessen.

## Patentansprüche

1. Vorrichtung zur Messung eines optischen Durchbruches, der in einem transparenten Gewebe (6, 14) unterhalb einer Gewebeoberfläche von einer Behandlungs-Laserstrahlung (2) ausgelöst wird, die eine laser-chirurgische Einrichtung (1) in einem im transparenten Gewebe (6, 14) liegenden Behandlungsfokus (11) bündelt, wobei die Vorrichtung einen Detektionsstrahlengang mit einer Optik aufweist, die Optik aus dem transparenten Gewebe (6, 14) unterhalb der Gewebeoberfläche ausgehende Strahlung in den Detektionsstrahlengang einkoppelt, wobei die Vorrichtung weiterhin eine Beleuchtungsstrahlungsquelle (5, 29, 53) umfasst, die Beleuchtungsstrahlung in das transparente Gewebe (14) einkoppelt, **dadurch gekennzeichnet, dass** die Beleuchtungsstrahlungsquelle (5) und der Detektionsstrahlengang Teil eines Interferometeraufbaus (3) sind, der als optischer Kohärenztomograph ausgebildet ist, welcher die aus dem transparenten Gewebe ausgehende Strahlung räumlich filtert, und dass dem Detektionsstrahlengang eine Detektoreinrichtung (4, 3, 9; 39, 40, 41; 58, 59, 60) nachgeordnet ist, die aus rückgestreuter Beleuchtungsstrahlung ein Detektionssignal erzeugt, das axiale Ausdehnung und/oder Lage des optischen Durchbruchs im transparenten Gewebe (6, 14) anzeigt.

2. Vorrichtung nach Anspruch 1, **dadurch gekennzeichnet, daß** die Beleuchtungsstrahlungsquelle auch zur Abgabe der Behandlungs-Laserstrahlung (2) ausgebildet ist.

3. Vorrichtung nach Anspruch 1, **dadurch gekennzeichnet, daß** der Interferometeraufbau (3) einen Meßarm und einen verstellbaren Referenzarm (7, 8) aufweist, wobei die Beleuchtungsstrahlung in Strahlrichtung eine Kohärenzlänge aufweist, von der die Auflösung abhängt, mit der das Detektionssignal die räumliche Ausdehnung anzeigt, wobei Interferenz nur auftritt, wenn Länge von Meßarm und Referenzarm sich maximal um die Kohärenzlänge unterscheiden.

4. Vorrichtung nach Anspruch 1 oder 3, **dadurch gekennzeichnet, daß** die Beleuchtungsstrahlungsquelle die Beleuchtungsstrahlung (12) in einen im Gewebe (14) gelegenen Beleuchtungsfokus (11) bündelt, wobei die Lage des Beleuchtungsfokusses (11) zur Erzeugung des Detektionssignals verstellbar ist.

5. Vorrichtung nach Anspruch 4, **dadurch gekennzeichnet, daß** die Beleuchtungsstrahlung in einen Lichtweg der Behandlungs-Laserstrahlung eingekoppelt ist, wobei eine verstellbare Optik (26) verwendet ist, mit der die Divergenz der Beleuchtungsstrahlung ohne Veränderung der Divergenz der Behandlungs-Laserstrahlung veränderbar ist.

6. Vorrichtung nach Anspruch 1 oder 2, **dadurch gekennzeichnet, daß** der Detektionstrahlengang (67) eine optische Achse (61) aufweist, die schräg zu einer optischen Achse (62, 60) der Behandlungs-Laserstrahlung oder der Beleuchtungsstrahlung liegt.

7. Vorrichtung nach einem der obigen Ansprüche, **dadurch gekennzeichnet, daß** die Detektoreinrichtung ein Maß für die räumliche Ausdehnung und/oder Lage einzelner, durch die Wechselwirkung erzeugter Streuzentren bestimmt, bevorzugt der Streuzentren in einer Augenhornhaut.

8. Vorrichtung nach einem der obigen Ansprüche, **dadurch gekennzeichnet, daß** das Detektionssignal (5) einen Durchmesser einer Plasmablase (11) anzeigt, die durch den optischen Durchbruch erzeugt wurde.

9. Vorrichtung nach einem der obigen Ansprüche, **gekennzeichnet durch** eine Scanvorrichtung (21, 54) zum Abscannen des Gewebes (14).

## Claims

1. A device for measuring an optical break-through which is created in a transparent tissue (6, 14), beneath a tissue surface by treatment laser radiation (2) which a laser surgical unit (1) focuses into a treatment focus (11), said focus being located in the transparent tissue (6, 14), wherein said device comprises a detection beam path including optics, wherein the optics couple radiation emitted from the transparent tissue (6, 14), from beneath the tissue surface, into the detection beam path, wherein the device further comprises a source of illumination radiation (5, 29, 53) which couples illumination radiation into the transparent tissue (14), **characterized in that** the source of illumination radiation (5) and the detection beam path are part of an interferometer structure (3) which is configured as optical coherence tomograph spatially filtering radiation extending from the transparent tissue and that a detector unit (4, 3, 9; 39, 40, 41; 58, 59, 60) is arranged following the detection beam path, said detector unit (4, 3, 9; 39, 40, 41; 58, 59, 60) generating a detection signal which indicates the axial extent and/or position of the optical break-through in the transparent tissue (6, 14).

2. The device according to claim 1, **characterized in that** the source of illumination radiation is also provided for emission of the treatment laser radiation (2).

3. The device according to claim 1, **characterized in that** the interferometer structure (3) comprises a measuring arm and an adjustable reference arm (7, 8), with the illumination radiation having a coherence length in the direction of beam propagation, on which coherence length the resolution depends at which the detection signal indicates the spatial extent, wherein interference occurs only, if the lengths of the measuring arm and of the reference arm differ by no more than the coherence length.

4. The device according to claim 1 or 3, **characterized in that** the source of illumination radiation focuses the illumination radiation (12) into an illumination focus (11) located in the tissue (14), wherein the position of the illumination focus (11) is adjustable for generating the detection signal.

5. The device according to claim 4, **characterized in that** the illumination radiation is coupled into a light path of the treatment laser radiation, wherein adjustable optics (26) are used by which the divergence of the illumination radiation is changeable without changing the divergence of the treatment laser radiation.

6. The device according to claim 1 or 2, **characterized in that** the detection beam path (67) comprises an optical axis (61) which is oblique to an optical axis (62, 60) of the treatment laser radiation or of the illumination radiation.

7. The device according to any of the above claims, **characterized in that** the detector unit determines a measure of the spatial extent and/or position of individual scattering centers generated by the interaction, preferably of the scattering centers in a cornea of an eye.

8. The device according to any of the above claims, **characterized in that** the detection signal (5) indicates a diameter of a plasma bubble (11), which was generated by the optical break-through.

9. The device according to any of the above claims, **characterized by** a scanning device (21, 54) for scanning the tissue (14).

## Revendications

1. Dispositif de mesure d'un percement optique déclenché dans un tissu transparent (6, 14), sous la surface du tissu, par un rayonnement laser de traitement (2) qui concentre un dispositif chirurgical laser (1) dans un foyer de traitement (11) situé dans le tissu transparent (6, 14), dans lequel le dispositif présente un trajet de faisceau de détection comportant une optique, l'optique injecte un rayonnement émanant du tissu transparent (6, 14) sous la surface du tissu dans le trajet du faisceau de détection, dans lequel le dispositif comprend en outre une source de rayonnement d'éclairage (5, 29, 53) injectant le rayonnement d'éclairage dans le tissu transparent (14), **caractérisé en ce que** la source de rayonnement d'éclairage (5) et le trajet du faisceau de détection font partie d'un montage interférométrique (3) qui est réalisé sous la forme d'un tomographe à cohérence optique filtrant spatialement le rayonnement émanant du tissu transparent, et **en ce qu'**un dispositif détecteur (4, 3, 9 ; 39, 40, 41 ; 58, 59, 60) est disposé en aval du trajet du faisceau de détection, lequel dispositif détecteur génère à partir du rayonnement d'éclairage rétrodiffusé un signal de détection qui indique l'extension axiale et/ou la position du percement optique dans le tissu transparent (6, 14).

2. Dispositif selon la revendication 1, **caractérisé en ce que** la source de rayonnement d'éclairage est également conçue pour délivrer le rayonnement laser de traitement (2).

3. Dispositif selon la revendication 1, **caractérisé en ce que** le montage interférométrique (3) comporte un bras de mesure et un bras de référence réglable (7, 8), dans lequel le rayonnement d'éclairage présente une longueur de cohérence, dans la direction de rayonnement, dont dépend la résolution avec laquelle le signal de détection indique l'extension spatiale, dans lequel l'interférence ne se produit que lorsque les longueurs du bras de mesure et du bras de référence diffèrent au maximum l'une de l'autre de la longueur de cohérence.

4. Dispositif selon la revendication 1 ou 3, **caractérisé en ce que** la source de rayonnement d'éclairage concentre le rayonnement d'éclairage (12) sur un foyer d'éclairage (11) situé dans le tissu (14), dans lequel la position du foyer d'éclairage (11) peut être réglée pour générer le signal de détection.

5. Dispositif selon la revendication 4, **caractérisé en ce que** le rayonnement d'éclairage est injecté sur un chemin optique du rayonnement laser de traitement, dans lequel une optique réglable (26) est utilisée au moyen de laquelle on peut modifier la divergence du rayonnement d'éclairage peut être modifiée sans modifier la divergence du rayonnement laser de traitement.

6. Dispositif selon la revendication 1 ou 2, **caractérisé en ce que** la trajectoire (67) du faisceau de détection présente un axe optique (61) qui est oblique par rapport à un axe optique (62, 60) du rayonnement laser de traitement ou du rayonnement d'éclairage.

7. Dispositif selon l'une quelconque des revendications précédentes, **caractérisé en ce que** le dispositif détecteur détermine une mesure de l'extension spatiale et/ou de la position de centres de diffusion individuels créés par l'interaction, de préférence les centres de diffusion se situant dans une cornée.

8. Dispositif selon l'une quelconque des revendications précédentes, **caractérisé en ce que** le signal de détection (5) indique un diamètre d'une bulle de plasma (11) qui a été créée par le percement optique.

9. Dispositif selon l'une quelconque des revendications précédentes, **caractérisé par** un dispositif de balayage (21, 54) destiné à explorer le tissu (14).
